# EUROPEAN PATENT SPECIFICATION

(11) **EP 0 569 830 B1**
(45) Date of publication and mention of the grant of the patent: **23.06.1999**
(21) Application number: 93107253.2
(22) Date of filing: 05.05.1993
(51) Int. Cl.: F17D 3/10, G01N 1/20

(54) **Method and valve for sampling milk and bottle for use in such sampling**
Verfahren und Ventil zur Probenahme von Milch und Flasche zu solcher Entnahme
Méthode et vanne pour le prélèvement de lait et bouteille pour tel prélèvement

(30) Priority: 13.05.1992 DK 62892
(43) Date of publication of application: 18.11.1993
(73) Proprietor: DANSK MEJERI ELEKTRONIK A/S, DK-8382 Hinnerup (DK)
(72) Inventor: Oestergaard, Hans, DK-8210 Aarhus V (DK)
(74) Representative: Nielsen, Leif

(56) References cited:
- CH-A- 544 302
- DE-A- 2 453 473
- DE-B- 1 065 187
- DE-U- 8 701 550
- US-A- 4 744 255

## Description

The present invention relates to a valve assembly, a system and a method for sampling milk by draining milk from a farm tank to a tank lorry.

Different systems for sampling milk are known. However, none of these samples have in a satisfying and secure way made it possible simultaneously to obtain a well-defined sample, which is distributed evenly over the entire amount of milk, which is drained from a farm tank to a tank lorry, at the same time that both the supplier and the dairy are certain that the sample has not been manipulated.

Two systems are used for draining farm tanks, viz. a vacuum system or a pressure system. With a vacuum system is meant a system, in which the tank of the tank lorry is subjected to a vacuum, thus a suction occurs through the drain pipe. With a pressure system is meant a system, in which a pump is inserted into the drain pipe so that the drain pipe contains a pressure side leading to the tank of the tank lorry and a suction side leading to the farm tank.

CH 544 302 discloses a valve-system with a pipe in which fluid from a chemical process is flowing and a discharge opening for discharging a sample of the fluid. The sample of fluid is collected by a piston with one or more grooves. The piston is displaced from a first position in which the grooves communicates with the discharge opening to a second position in which the the grooves communicate with the fluid in the pipe. The piston is then displaced back to the first position for discharging fluid collected in the grooves.

This valve-system, however, has some deficiencies. The fact that the piston is displaced from a position out of the flow of fluid to a position into the flow means that the flow is disturbed when the piston suddenly enters the flow of fluid. The sample collected by the grooves is therefore not a representative sample. Furthermore, the grooves are dimensioned so that suspensions and solids being present in the fluid will not enter the discharge opening. Furthermore, this means that the sample collected is not a representative sample.

It is the object of the present invention to remedy the drawback of the prior art, and which moreover makes a consistent and representative and reliable sampling possible, irrespective of a vacuum system or a pressure system is used.

According to the present invention this is obtained by a valve assembly for sampling milk by draining milk from a farm tank to a tank lorry comprising a valve, which is connected with a drain pipe, the sampling valve comprises a cylindrical piston, which on its surface is provided with an annular groove, which defines a given capacity within the cylinder surface of the piston, said groove is positioned on the piston in such a way that in the first outer position of the piston it is in connection with the drain pipe, and in the second outer position of the piston it is in connection with the discharge opening of the sampling valve, characterized in that the piston is positioned concentrically around an axis extending through the discharge opening, that a cylindrical lining delimiting the radial extension of the groove is provided between the two outer positions of the piston, that the discharge opening of the valve casing is connected with a passage extending substantially perpendicular to the axis through the piston and the discharge opening, that the discharge opening of the sampling valve is provided with a truncated cone-shaped profile corresponding to the profile for a collar on the neck of a sample bottle secured in the discharge opening, that the valve assembly furthermore comprises reciprocal means and a displaceable plate, which has a recess made for engaging in an annular groove below a collar of the neck of said bottle, and which is connected with the reciprocal means which in one outer position presses the profiled collar of the neck of said bottle into sealing engagement with the corresponding profiled discharge opening during the sampling, and that the valve assembly comprises a control and data processing unit, which controls the functions of the valve assembly depending on the amount of the sample and the amount of milk to be drained.

Also a system comprising such valve assembly is disclosed. Said system is provided in combination with a sampling bottle, whereby at least the neck of the bottle is made from a deformable material, preferably of plastic, that the neck of the bottle is provided with a profiled collar, which is made for sealing engagement in the discharge opening of the valve, and that below the collar of the bottle an annular groove is formed, which is dimensioned for receiving the plate with which the bottle is displaced against the discharge opening and is secured during the sampling.

Moreover, a method for use of this valve assembly is disclosed, viz. a method for sampling milk by draining milk from a farm tank to a tank lorry and comprising that a sample bottle is inserted into the valve assembly as it is connected with the discharge opening of the sampling valve in which it is secured during the sampling, that the sample is taken by means of the cylindrical piston, which in its cylinder surface has an annular groove, which within the cylinder surface defines a given capacity, wherein the piston is displaced a number of times defined in advance between a first position, in which the annular groove is connected with the drain pipe past a cylindrical lining, which delimits the radial extension of the annular groove, to a second position, in which the annular groove is connected with the discharge opening of the valve for supplying milk into said bottle, wherein the number of piston strokes is evenly distributed over the entire draining and is determined by the control and data processing unit based on pre-entered data concerning the actual content of the farm tank, and wherein said bottle subsequently is released automatically from its securing in the valve assembly as a result of a signal from the control and data processing unit.

With such a valve assembly and such a method for its use it is possible by means of the control and data processing unit to drain a well-defined milk capacity each time the piston makes a travel. The reciprocal movement of the piston may be distributed evenly over the entire cyclus of draining the farm tank. Consequently, a representative sample is obtained.

The method makes a precise sample possible, irrespective of a vacuum system or a pressure system is used, and irrespective of the sample being taken at the first supplier, where the tank lorry is empty, or at a final supplier, where the tank lorry is approximately entirely full. This is possible as the sampling only is defined by the encircling capacity, which is located in the annular groove of the piston and the number of reciprocal movements which the piston carries out.

As a sample bottle is secured in the valve assembly during the entire sampling, a reliable sample is obtained.

The supplier and the dairy may attain security that an unintended or inadmissible manipulation with the sample has not occurred as a bottle may be sealed in advance while it is sterilized. After the piston with its first stroke has broken through a film or the membrane covering the opening of the neck of said bottle, the bottle can be filled. When said bottle is full it may only be removed from the sampling valve after it is closed by pressing a ball in the neck of said bottle. If the neck of said bottle is deformed by the pressing of the ball, the ball may only be removed by cutting off the neck. This precludes unintended or inadmissible manipulation of a sample.

With a system according to claim 3 the suspension of the bottle and the securing may occur in a very simple way, only by using a plate provided with a recess, which engages in the annular groove of the neck of the bottle. By placing the bottle in the recess of the plate a reliable positioning of said bottle is obtained in relation to the discharge opening of the valve so that a subsequent placing in sealing engagement is easily obtained. By forming the collar like a truncated cone-shaped solid shell, partly a wedging action is obtained which ensures a correct placing in the discharge opening, at the same time as a large support surface is obtained, which makes a reliable securing possible with said bottle pressed against the discharge opening.

The invention will now be further explained with reference to the accompanying drawing, wherein
- Fig. 1: shows a view for illustrating a system, in which the method is used and in which the valve assembly according to the invention are included,
- Fig. 2: shows a section through a first embodiment of a valve assembly according to the invention,
- Fig. 3: shows a section through a second embodiment of a valve assembly according to the invention, seen from one side,
- Fig. 4: shows a section through the valve assembly shown in Fig. 3, seen according to IV-IV in Fig. 3,
- Fig. 5: shows a partial section view for illustrating a bottle suspended in a plate, which is intended for being connected with the valve assembly shown in Figs. 3 and 4, and
- Fig. 6: shows a view, from above, of the valve assembly shown in Fig. 3.

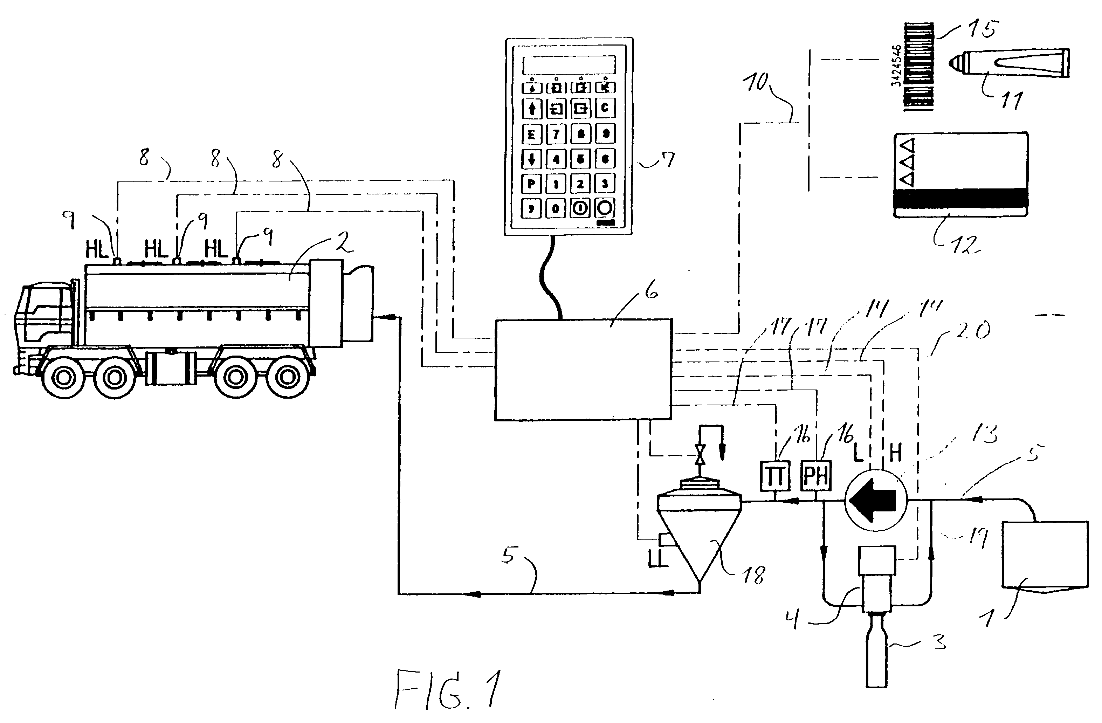

Figure 1 shows a valve assembly for sampling milk by a method according to the invention. 1 indicates a farm tank, 2 indicates a tank lorry, 3 indicates a sample bottle, 4 indicates a sampling valve, and 5 indicates a drain pipe, which transports the milk from the farm tank 1 to the tank lorry 2. The valve assembly comprises a control and data processing unit 6, which is connected with a keyboard 7. The unit 6 is via pipes 8 connected with sensors 9 on the tank lorry 2 in order to monitor the level in the tank. The unit 6 is via a pipe 10 connected with a bar code reader 11 for reading a bar code 15, which is positioned on the sample bottle 3. The unit 6 is also made for recording a magnetic card 12, which is individual for each and single supplier. Consequently, the unit 6 is given information about the individual supplier just as it is ensured that the sample bottle in question belongs to the supplier in question. The valve assembly may via the keyboard 7 or via the magnetic card 12 be provided with information about the amount of milk to be fetched from the farm tank 1.

The valve assembly shown is intended for use in a pressure system and comprises a pump 13, which is inserted in the drain pipe 5. The pump 13 is via the control wires 14 connected with the unit 6. The drain pipe 5 is provided with sensors 16, which measure the different parametres in the milk, and transmit the signals about this to the unit 6 via wires 17. A wellknown air trap 18 is inserted in the drain pipe 5.

The sampling valve 4 is inserted into a branching pipe 19 parallel to the pump 13. Thus, the branching pipe 19 connects the pressure side of the pump and the suction side of the pump for establishing a flow through the valve 4. The valve 4 is via a control wire 20 connected with the unit 6.

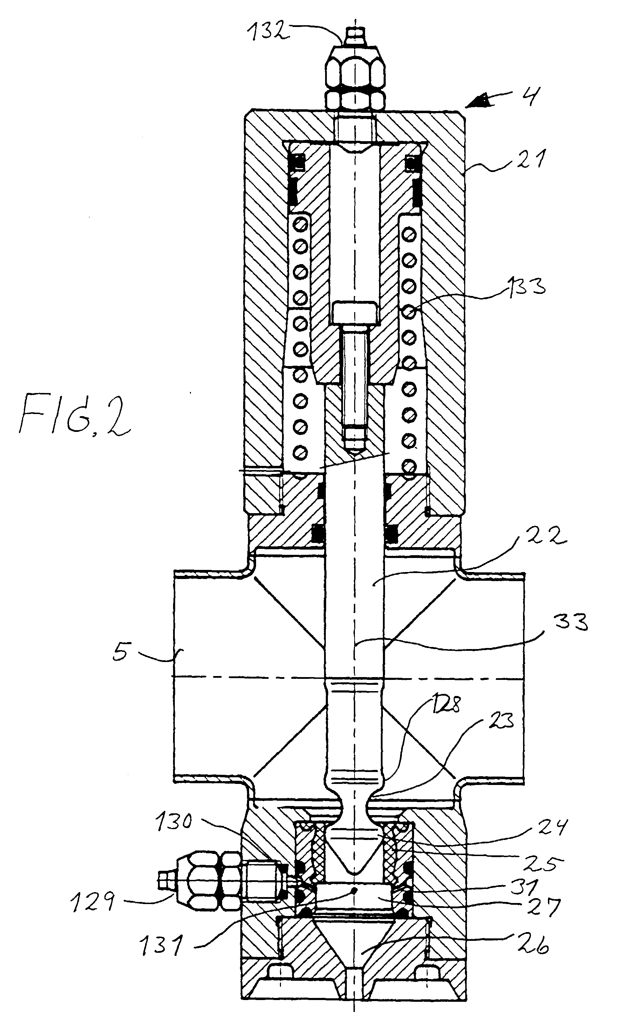

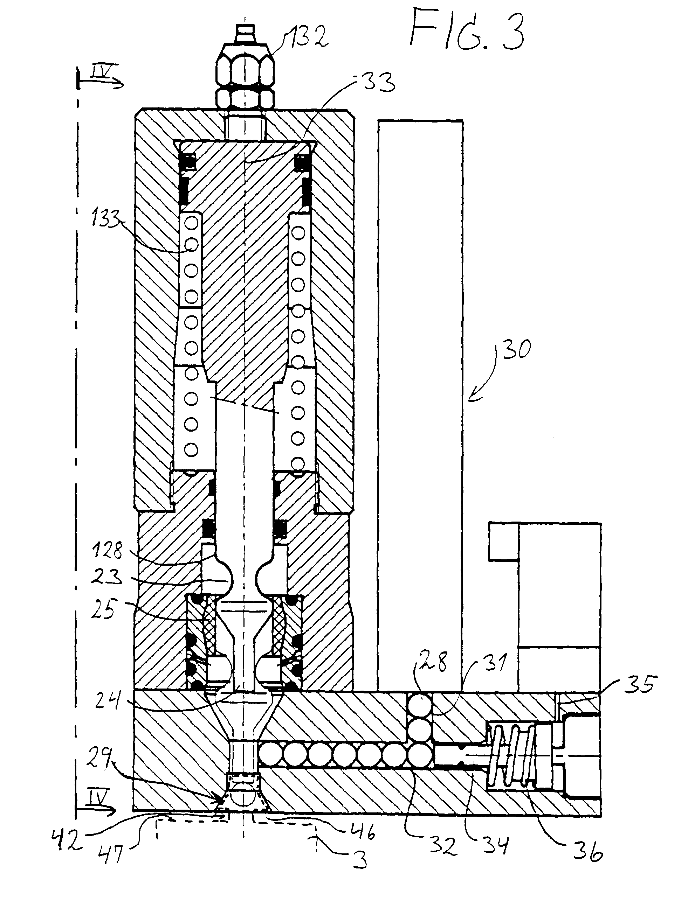

The valve 4 shown schematically in Figure 1 may be of the type illustrated in Figure 2 or of the type illustrated in Figures 3-6.

Figure 2 illustrates a first embodiment of a valve 4. The valve comprises a valve casing 21, in which a cylinder piston 22 may be displaced backwards and forwards for a reciprocal movement. The piston 22 is provided with an annular groove 23 in the vicinity of its free end. The piston 22 passes through the drain pipe 5, which may be intended for a vacuum system or a pressure system as illustrated in Figure 1. The piston 22 has such a travel that the lowest end 24, which contains the annular groove 23, may be displaced past a lining 25, which is located between the drain pipe 5 and a discharge opening 26 for the valve 4. By the reciprocal movement of the piston the annular groove 23 may be cut off completely by the lining 25. Thus, by a downwardly directed movement in relation to the shown position, the piston may be led all the way down to a chamber 27 in connection with the discharge opening 26. Consequently, the milk may now run freely out of the discharge opening, irrespective of the drain pipe 5 being subjected to vacuum or to pressure. This is possible as the annular groove 23 is disconnected with the drain pipe 5. In the embodiment shown the piston 22 has, in an area 128 adjacent to the annular groove, a diameter which fits tightly in the diameter of the lining 24.

When the piston 22 has carried out a desired number of movements and has drained a desired amount of milk to a sample bottle, which is connected with the discharge opening 26 of the valve, the valve may be blown clean by means of an air supply 129, which via an annular passage 130 and radial passages 131 is in connection with the chamber 27. By inserting a sample bottle milk leftovers from a previous supplier will not exist.

The piston 22 in the valve 4 is operated pneumatically and is activated for a downwardly directed stroke by air supply via an inlet connecting piece 132. The return movement of the piston is established by means of a spring 133. It is noted that alternative methods for operating the piston 22 are possible. It is solely to be ensured that the piston in its first outer position as illustrated in Figure 2, is in connection with the drain pipe 5, and that the annular groove 23 in the second outer position of the piston is in connection with the discharge opening 26.

Figures 3-6 illustrate an alternative embodiment of a valve assembly according to the invention. As several parts are repeated in the two embodiments, only a specific explanation will be given of the elements, which differentiate the two embodiments.

This valve assembly may also be used in a pressure system or a vacuum system. The situation illustrated corresponds to Figure 1 as the valve is not inserted directly via a drain pipe 5 as shown in Figure 2. The valve 4 is connected with a branching wire 19, which because of the pump 13 fills a chamber 34 with milk. This chamber will be in connection with the annular groove 23 in the first outer position of the piston 22, which is shown with full-drawn lines. The annular groove 23 has an axial extension which also here, is less than the axial extension of the lining 25. Thus, the groove 23 may, at its downwardly directed stroke towards the second outer position which is shown with thin lines, pass by the lining 25, which delimits a well-defined capacity in the groove 23. When the piston is located in its second position the annular groove 23 will, as illustrated with thin lines, be in connection with the chamber 27 above the discharge opening 26. Consequently, the milk may run down into the sample bottle 3.

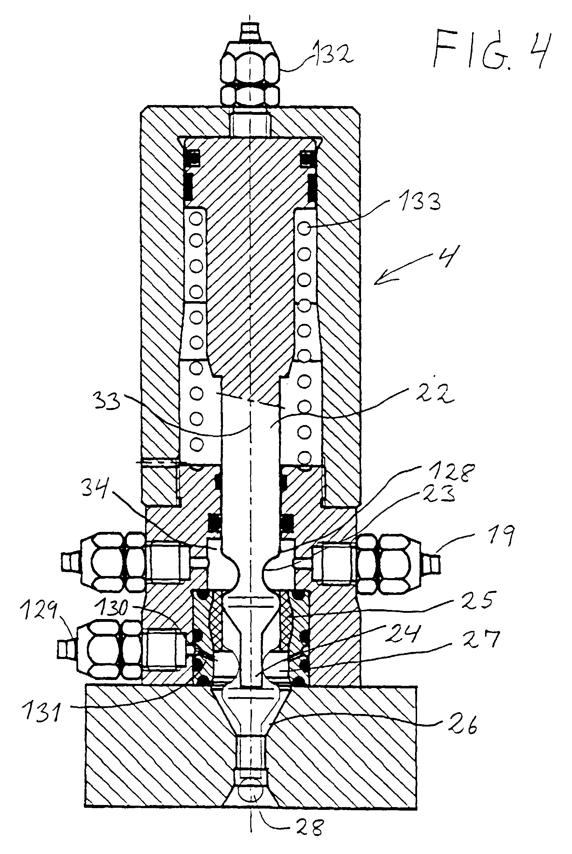

Figures 3 and 4 illustrate the final stroke of the piston 22, where a ball 28 has been pressed into the neck 29 of the bottle 3. The ball comes from a storage 30 for balls, which is connected with the valve 4. From the bottom of the storage 30 for balls, balls are led through a vertical passage 31 to a horizontal passage 32, which is located substantially perpendicular to an axis 33, which extends through the piston 22 and the neck 29 of the bottle. In the passage 32 a row of balls are placed, or merely a single ball 28, depending on the length of the passage 32. At the end of the passage 32 a piston 34 is positioned, which by activating through a compressed air pipe 35, may press a ball into the discharge opening 26 beneath the outermost end part 24 of the piston 22. The piston 34 is returned by means of a spring 36. The piston 34 is activated as a result of a signal from the unit 6.

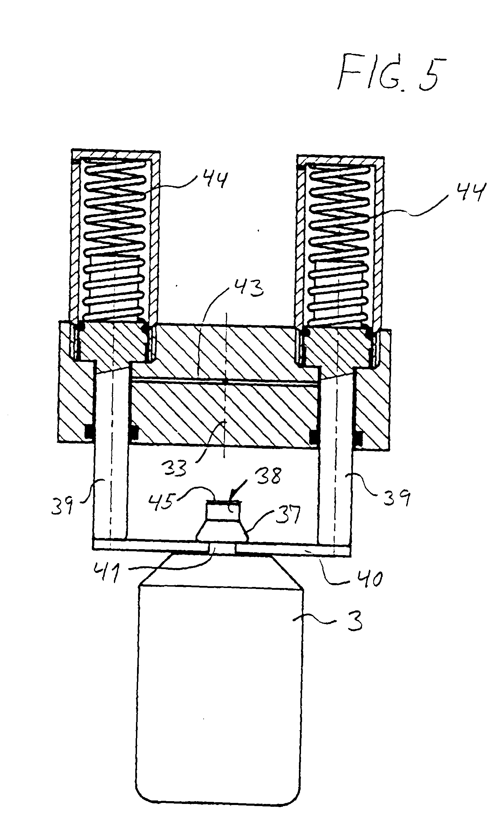

The balls 28 are preferably steel balls, which have an oversize in relation to the diameter of the neck 29 of the bottle, which is of plastic. After the pressing, the ball will thus deform the neck of the bottle. Because of the form of the neck of the bottle having a collar in the form of an annular shell 37 (Figure 5), there is no risk of breaking the neck of the bottle.

As the outermost end 38 of the neck of the bottle is funnel-shaped, there will be no difficulties about ensuring the placing of the ball 28 in the neck 29 of the bottle. The truncated cone-shaped collar 37 of the neck of the bottle fits into the outermost part of the discharge opening 26, which is formed with a profile corresponding to the collar 37.

Figure 5 illustrates two pistons 39, which support a plate 40, provided with a recess 41. The bottle 3 may be positioned in the recess 41, and will be aligned with the discharge opening 26 as an annular groove 42 (see Figure 4), which is formed below the collar 37, is positioned in the recess 41. The pistons 39 are subsequently activated by compressed air via a passage 43. Consequently, the neck 29 of the bottle is pressed up into the discharge opening 26 in a sealing engagement. The return movement of the pistons 39 is ensured by means of springs 44.

The bottle 3 is provided with a membrane 45, which covers the opening of the neck of the bottle, and which will be broken by the first piston stroke as the outermost end part 24 of the piston 22 penetrates into the neck of the bottle. The bottle 3 will be secured during the entire filling and during the above described sealing with the ball 29. As the entire process is controlled and monitored by the unit 6, an unintended or inadmissible manipulating with the sampling will be prevented.

As the annular groove of the bottle is delimited by a plane under side 46 of the collar and a plane top side 47 of the bottle 3, the engaging mean may quite simply be constituted of the plane plate 40, in which the bottle 3 in a safe way is suspended in the recess 41. As the collar 37 is truncated cone-shaped a large support surface 46 is obtained, which rests on the top side of the plate 40. This gives a stable support so the bottle does not turn over when it is pressed into the discharge opening 26. The large support surface is simultaneously advantageous for a safe securing of the bottle below the pressing of the ball 28 into the neck of the bottle. Consequently, the ball may be pressed with great force which causes a deforming of the neck of the bottle. As the sample bottle 3 is made of plastic, the material in the neck will resiliently be restored to its original position so that the ball cannot be removed. By the subsequent sampling of the sample bottle 3 in the dairy, the neck may quite simply be cut off together with the steel ball by cutting in the bottom of the annular groove 42.

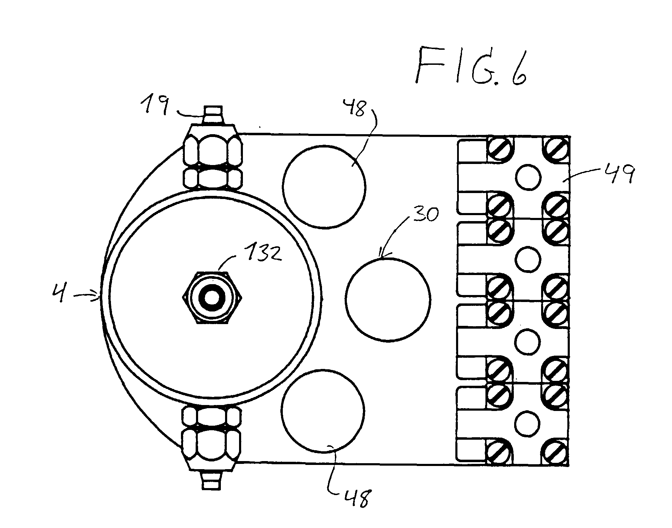

Figure 6 illustrates the valve assembly seen from above. Thus, the placing of the storage 30 for balls and the placing of two casings 48 are seen, said casings containing the pistons 39 and the springs 44. Furthermore, a connection arrangement 49 is shown through which the valve 4 is connected with the unit 6.

## Claims

1. Valve assembly for sampling milk by draining milk from a farm tank (1) to a tank lorry (2) comprising a valve (4), which is connected with a drain pipe (5), the sampling valve comprises a cylindrical piston (22), which on its surface is provided with an annular groove (23), which defines a given capacity within the cylinder surface of the piston, said groove (23) is positioned on the piston (22) in such a way that in the first outer position of the piston it is in connection with the drain pipe (5) , and in the second outer position of the piston it is in connection with the discharge opening (26) of the sampling valve (9), **characterized** in that the piston (22) is positioned concentrically around an axis (33) extending through the discharge opening (26), that a cylindrical lining (25) delimiting the radial extension of the groove (23) is provided between the two outer positions of the piston, that the discharge opening (26) of the valve (4) casing is connected with a passage extending substantially perpendicular to the axis (33) through the piston (22) and the discharge opening (26), that the discharge opening (26) of the sampling valve (4) is provided with a truncated cone-shaped profile corresponding to the profile for a collar (37) on the neck (29) of a sample bottle (3) secured in the discharge opening, that the valve assembly furthermore comprises reciprocal means (39,44) and a displaceable plate (40), which has a recess (41) made for engaging in an annular groove (42) below a collar (37) of the neck (29) of said bottle (3), and which is connected with the reciprocal means (39,44) which in one outer position presses the profiled collar (37) of the neck of said bottle into sealing engagement with the corresponding profiled discharge opening (26) during the sampling, and that the valve assembly comprises a control and data processing unit (6), which controls the functions of the valve assembly depending on the amount of the sample and the amount of milk to be drained.

2. Valve assembly according to claim 1, **characterized** in that the passage is connected with a storage (30) for balls (28), which is provided with a ball advancing unit (34), which is made for advancing a ball (28) into the path of the movement of the piston (22).

3. A system for sampling milk comprising a valve assembly according to claim 1 in combination with a sampling bottle, whereby at least the neck of the bottle is made from a deformable material, preferably of plastic, that the neck of the bottle is provided with a profiled collar, which is made for sealing engagement in the discharge opening of the valve, and that below the collar of the bottle an annular groove is formed, which is dimensioned for receiving the plate with which the bottle is displaced against the discharge opening and is secured during the sampling.

4. Method for use of the valve assembly according to claim 1 or 2 for sampling milk by draining milk from a farm tank (1) to a tank lorry (2) and comprising that a sample bottle (3) is inserted into the valve assembly as it is connected with the discharge opening (26) of the sampling valve (4) in which it is secured during the sampling, that the sample is taken by means of the cylindrical piston (22), which in its cylinder surface has an annular groove (23), which within the cylinder surface defines a given capacity, wherein the piston (22) is displaced a number of times defined in advance between a first position, in which the annular groove (23) is connected with the drain pipe (5) past a cylindrical lining (25), which delimits the radial extension of the annular groove (23), to a second position, in which the annular groove (23) is connected with the discharge opening (26) of the valve (4) for supplying milk into said bottle (3), wherein the number of piston strokes is evenly distributed over the entire draining and is determined by the control and data processing unit (6) based on pre-entered data concerning the actual content of the farm tank (1), and wherein said bottle (3) subsequently is released automatically from its securing in the valve assembly as a result of a signal from the control and data processing unit (6).

5. Method according to claim 4, **characterized** in that after removal of said bottle the sampling valve is blown clean automatically with compressed air.

## Patentansprüche

1. Ventileinheit zur Probenahme von Milch durch Leiten der Milch aus einem Tank (1) eines Bauernhofs zu einem Tankwagen (2), aufweisend ein Ventil (4), das mit einem Ableitungsrohr (5) verbunden ist, wobei das Probenahmeventil einen zylindrischen Kolben (22) aufweist, der auf seiner Oberfläche mit einer ringförmigen Nut (23) versehen ist, die ein gegebenes Fassungsvermögen innerhalb der zylindrischen Oberfläche des Kolbens definiert, wobei die Nut (23) so auf dem Kolben (22) positioniert ist, daß sie in der ersten äußeren Position des Kolbens mit dem Ableitungsrohr (5) in Verbindung ist und in der zweiten äußeren Position des Kolbens mit der Entleerungsöffnung (26) des Probenahmeventils (9) in Verbindung ist, dadurch gekennzeichnet, daß der Kolben (22) konzentrisch um eine Achse (33) positioniert ist, die sich durch die Entleerungsöffnung (26) erstreckt, daß eine zylindrische Auskleidung (25), die die radiale Ausdehnung der Nut (23) begrenzt, zwischen den beiden äußeren Positionen des Kolbens vorgesehen ist, daß die Entleerungsöffnung (26) des Ventil-(4)Gehäuses mit einem Kanal verbunden ist, der sich im wesentlichen senkrecht zur Achse (33) durch den Kolben (22) und die Entleerungsöffnung (26) erstreckt, daß die Entleerungsöffnung (26) des Probenahmeventils (4) mit einem kegelstumpfförmigen Profil ausgebildet ist, das dem Profil für einen Kragen (37) auf dem Hals (29) einer Probenahmeflasche (3) entspricht, die in der Entleerungsöffnung gesichert ist, daß die Ventileinheit darüber hinaus hin- und hergehende Mittel (39, 44) und eine verschiebbare Platte (40) aufweist, welche eine Ausnehmung (41) für den Eingriff in eine ringförmige Nut (42) unterhalb eines Kragens (37) des Halses (29) der Flasche (3) aufweist und mit den hin- und hergehenden Mitteln (39, 44) verbunden ist, das in einer äußeren Position den profilierten Kragen (37) des Halses der Flasche in einen abdichtenden Eingriff mit der mit einem entsprechenden Profil versehenen Entleerungsöffnung (26) während der Probenahme drückt, und daß die Ventileinheit eine Kontroll- und Datenverarbeitungseinheit (6) aufweist, die die Funktionen der Ventileinheit in Abhängigkeit von der Probemenge und der Menge an abzuleitender Milch kontrolliert.

2. Ventileinheit nach Anspruch 1, dadurch gekennzeichnet, daß der Kanal mit einem Lager (30) für Kugeln (28) verbunden ist, das mit einer Kugelvorschubeinheit (34) versehen ist, die dafür gemacht ist, eine Kugel (28) in den Weg der Bewegung des Kolbens (22) vorzuschieben.

3. System zur Probenahme von Milch, aufweisend eine Ventileinheit nach Anspruch 1 zusammen mit einer Probenahmeflasche, wobei zumindest der Hals der Flasche aus einem deformierbaren Material, vorzugsweise aus Kunststoff, gemacht ist, daß der Hals der Flasche mit einem profilierten Kragen versehen ist, der für einen abdichtenden Eingriff in die Entleerungsöffnung des Ventils gemacht ist, und daß unterhalb des Kragens der Flasche eine ringförmige Nut ausgebildet ist, die für die Aufnahme der Platte dimensioniert ist, mit der die Flasche gegen die Entleerungsöffnung verschoben wird und während der Probenahme gesichert wird.

4. Verfahren zur Verwendung der Ventileinheit nach Anspruch 1 oder 2 zur Probenahme von Milch durch Ableiten von Milch aus einem Tank (1) eines Bauernhofs zu einem Tankwagen (2) hin, wobei eine Probenahmeflasche (3) in die Ventileinheit eingeschoben wird, wenn sie mit der Entleerungsöffnung (26) des Probenahmeventils (4), in der sie während der Probenahme gesichert ist, verbunden wird, wobei die Probe mittels eines zylindrischen Kolbens (22) genommen wird, der in seiner zylindrischen Oberfläche eine ringförmige Nut (23) aufweist, die innerhalb der Zylinderoberfläche ein vorgegebenes Fassungsvermögen definiert, wobei der Kolben (22) eine Anzahl von zuvor definierten Malen zwischen einer ersten Position, in der die ringförmige Nut (23) mit dem Ableitungsrohr (5) verbunden ist, über eine zylindrische Auskleidung (25), die die radiale Ausdehnung der ringförmigen Nut (23) begrenzt, zu einer zweiten Position verschoben wird, in der die ringförmige Nut (23) mit der Entleerungsöffnung (26) des Ventils (4) verbunden ist, um der Flasche (3) Milch zuzuführen, wobei die Anzahl von Kolbenstößen gleichmäßig über den gesamten Ablaßvorgang verteilt ist und von der Kontroll- und Datenverarbeitungseinheit (6) auf zuvor eingegebenen Daten basierend, welche den aktuellen Inhalt des Tanks (1) des Bauernhofes betreffen, bestimmt wird und wobei die Flasche (3) anschließend automatisch aus ihrer Sicherung in der Ventileinheit als Ergebnis eines Signals von der Kontroll- und Datenverarbeitungseinheit (6) entkoppelt wird.

5. Verfahren nach Anspruch 4, dadurch gekennzeichnet, daß das Probenahmeventil nach der Entfernung der Flasche automatisch mit Druckluft sauber geblasen wird.

## Revendications

1. Assemblage de vanne pour le prélèvement de lait en laissant écouler du lait d'un tank de lait de ferme (1) à un camion-citerne (2), comprenant une vanne (4), liée à un tuyau purgeur (5), ledit assemblage de vanne comprenant un piston cylindrique (22) qui sur sa surface est muni d'une rainure annulaire (23), définissant une capacité déterminée à l'intérieur de la surface cylindrique du piston, ladite rainure (23) étant positionnée sur le piston (22) de telle façon que dans la première position extrême du piston, elle est en liaison avec le tuyau purgeur (5) et que dans la seconde position extrême du piston, elle est en liaison avec l'ouverture d'écoulement (26) de la vanne de prélèvement (9), **caractérisé** en ce que le piston (22) est placé de manière concentrique autour d'un axe (33) s'étendant à travers l'ouverture d'écoulement (26), qu'un revêtement cylindrique (25) délimitant l'extension radiale de la rainure (23) est pourvu entre les deux positions extrêmes du piston, que l'ouverture d'écoulement (26) de la boîte de la vanne (4) est liée à un passage s'étendant sensiblement perpendiculairement à l'axe (33) traversant le piston (22) et l'ouverture d'écoulement (26), que l'ouverture d'écoulement (26) de la vanne (4) de prélèvement est munie d'une section conique correspondant à la section d'une bague (37) du col (29) d'une bouteille de prélèvement (3) fixée dans l'ouverture d'écoulement, que l'assemblage de vanne en outre comprend des moyens réciproques (39,44) et un plateau déplaçable (40) ayant un découpage (41) fait pour s'engrener dans une rainure annulaire (42) sous une bague (37) du col (29) de ladite bouteille (3) et étant lié aux moyens réciproques (39, 44) qui dans une position extrême poussent la bague de section (37) du col de ladite bouteille vers l'engagement scellé avec l'ouverture d'écoulement (26) de section correspondante lors du prélèvement, et que l'assemblage de vanne comporte une unité de contrôle et de traitement de données (6) qui contrôle les fonctions de l'assemblage de la vanne en fonction de la quantité de prélèvement et de la quantité du lait à tirer.

2. Assemblage de vanne selon la revendication 1, **caractérisé** en ce que le passage est lié à un stockage (30) de boules (28) qui est muni d'un dispositif d'avancement de boules (34), prevu pour faire avancer une boule (28) dans le passage du mouvement du piston (22).

3. Un système de prélèvement de lait comprenant un assemblage de vanne selon la revendication 1 conjointement avec une bouteille de prélèvement dont au moins le col de la bouteille est fabriqué dans une matérière déformable, de préférence de plastique, **caractérisé** en ce que le col de la bouteille est muni d'une bague profilée destinée à l'engagement scellé de fermeture dans l'ouverture d'écoulement de la vanne et qu'en dessous de la bague de la bouteille, une rainure annulaire est formée qui est dimensionnée de sorte à recevoir le plateau avec lequel la bouteille est déplacée vers l'ouverture d'écoulement et est fixée lors du prélèvement.

4. Méthode pour l'emploi de la vanne selon les revendications 1 ou 2 pour le prélèvement de lait en tirant du lait d'un tank de lait de ferme (1) à un camion-citerne (2) et comprenant qu'une bouteille de prélèvement (3) est inserée dans l'assemblage de vanne (4) vu qu'elle est fixée lors du prélèvement, **caractérisée** en ce que le prélèvement est pris par l'intermédiaire du piston cylindrique (22) qui sur sa surface de cylindre a une rainure annulaire (23) qui à l'intérieur de la surface du cylindre définit une capacité déterminée, et dans lequel le piston (22) est déplacé un nombre de fois déterminé à l'avance entre une première position, dans laquelle la rainure annulaire (23) est liée au tuyau purgeur (5) après un revêtement cylindrique (25) qui délimite l'extension radiale de la rainure annulaire (23), à une seconde position dans laquelle la rainure annulaire (23) est liée à l'ouverture d'écoulement (26) de la vanne pour fournir du lait dans ladite bouteille (3), et dans lequel le nombre de tours du piston est régulièrement réparti sur tout le tirage et est déterminé par l'unité de contrôle et de traitement de données (6) sur la base des données préalablement introduites concernant le contenu actuel du tank de lait de ferme (1), et dans lequel ladite bouteille (3) par la suite est dégagée de sa fixation dans l'assemblage de vanne comme résultat d'un signal venant de l'unité de contrôle et de traitement de données (6).

5. Méthode selon la revendication 4, **caractérisée** en ce qu'après le dégagement de ladite bouteille, la vanne de prélèvement est soufflée propre avec de l'air comprimé.
